(19) Europäisches Patentamt
European Patent Office
Office européen des brevets

(11) Numéro de publication: **0 238 411**
**A1**

(12) # DEMANDE DE BREVET EUROPEEN

(21) Numéro de dépôt: **87400594.5**

(22) Date de dépôt: **17.03.87**

(51) Int.Cl.³: **C 07 D 471/04**
//(C07D471/04, 221:00, 209:00)

(30) Priorité: **17.03.86 FR 8604201**
**24.10.86 FR 8615290**

(43) Date de publication de la demande:
**23.09.87 Bulletin 87/39**

(84) Etats contractants désignés:
**AT BE CH DE ES FR GB GR IT LI LU NL SE**

(71) Demandeur: **SANOFI**
**40, Avenue George V**
**F-75008 Paris(FR)**

(71) Demandeur: **CENTRE NATIONAL DE LA RECHERCHE SCIENTIFIQUE (CNRS)**
**15, Quai Anatole France**
**F-75007 Paris(FR)**

(72) Inventeur: **Bisagni, Emile**
**16, rue Bossuet**
**F-91400 Orsay(FR)**

(72) Inventeur: **Chi Hung, Nguyen**
**7, allée des Amonts**
**F-91940 Les Ulis(FR)**

(72) Inventeur: **De Cointet, Paul**
**224 avenue Jean-Rieux**
**F-31500 Toulouse(FR)**

(74) Mandataire: **Polus, Camille et al,**
**c/o Cabinet Lavoix 2, Place d'Estienne d'Orves**
**F-75441 Paris Cedex 09(FR)**

(54) **Dihydro-1,2 méthyl-4 oxo-1 5-H pyrido (4,3-b) indoles et leur procédé de synthèse.**

(57) La présente invention est relative à de nouveaux composés qui sont des dihydro-1, 2 méthyl-4 oxo-1 5–H pyrido (4, 3–b) indoles de formule (I)

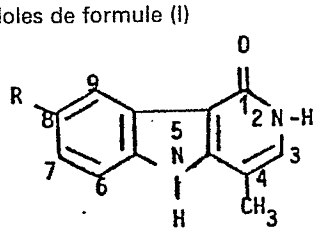

(I)

dans laquelle R représente l'hydrogène ou un groupe alkoxy ou arylcarbonyloxy. Ces composés sont des intermédiaries de synthèse dans la préparation de composés utilisables dans l'industrie pharmaceutique. L'invention concerne aussi leur synthèse.

EP 0 238 411 A1

L'invention est relative à de nouveaux composés qui sont des dihydro-1,2 méthyl-4 oxo-1 5-H pyrido (4,3-b) indoles de formule (I) suivante :

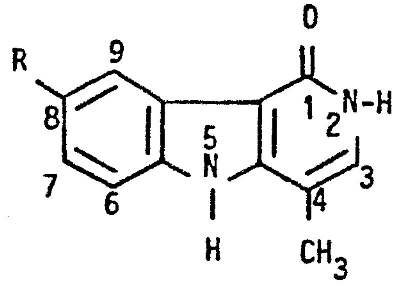

(I)

dans laquelle R représente l'hydrogène ou un groupe alkoxy en $C_1$-$C_4$ linéaire ou ramifié ou aryl($C_6$-$C_{10}$)carbonyloxy. Ces dérivés sont des intermédiaires dans la préparation des composés de formule (A) :

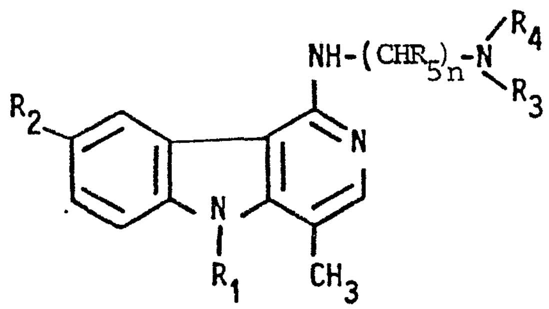

(A)

dans laquelle n représente un nombre entier de 2 à 4, $R_1$ représente l'hydrogène ou un groupe alkyle en $C_1$- $C_4$, $R_2$ représente l'hydrogène ou un groupe hydroxy ou un groupe alkoxy en $C_1$-$C_4$ , $R_3$ et $R_4$ sont chacun indépendamment l'un de l'autre l'hydrogène, un groupe alkyle en $C_1$-$C_4$ ou un groupe hydroxyalkyle, $R_5$ peut être identique ou différent et représente l'hydrogène, un groupe alkyle en $C_1$-$C_4$, ou un groupe hydroxy. Ces composés (A), qui sont doués de propriétés antitumorales intéressantes, font l'objet de la demande déposée ce même jour par les demandeurs et revendiquant la priorité de la demande de brevet français N° 86.04.202 du 17 mars 1986.

Le dihydro-1,2 oxo-1 5-H pyrido (4,3-b) indole et certains de ses dérivés ont été décrits antérieurement dans la littérature, notamment par CH. S. LEE et al. (Heterocycles, 16, 1081-1084, (1981)) et par C. DUCROCQ et al. (J. Heterocycl. Chem., 12, 963-967, (1975)).

Mais ces dérivés, dont la synthèse est différente du procédé objet de l'invention, ne comportent ni de groupes méthyle en position 4 ni de substituant en position 8 de telle sorte qu'ils ne peuvent pas être utilisés en tant qu'intermédiaires dans la préparation des composés de formule (A).

En outre, la méthode de préparation des composés de formule (I) utilisée dans la demande de brevet parallèle déposée ce même jour est celle décrite par E. BISAGNI et al. (Synthesis, 1984, 765). Cette méthode est caractérisée en ce qu'une paraalkyloxy phénylhydrazine en fort excès réagit sur la dihydro-1,2 hydroxy-4 méthyl-5 oxo-2 pyridine à haute température pour conduire aux composés de formule (I). Mais quelles que soient les conditions opératoires mises en oeuvre, le rendement en alkyloxy-8 dihydro-1,2 méthyl-4 oxo-2 5-H pyrido (4,3-b) indole (I) reste toujours inférieur à 20 % par rapport à l'oxopyridine. Or, le nouveau procédé de préparation, objet de l'invention, permet d'accéder aux composés de formule (I) avec un rendement supérieur à 50 % par rapport à l'oxopyridine. Ce nouveau procédé est caractérisé en ce que :

1) par réaction de la dihydro-1,2 hydrazino-4 méthyl-5 oxo-2 pyridine, (préparée selon la méthode décrite par E. BISAGNI, demande de brevet français N° 85.08.871 du 22 mars 1985, avec un rendement de 76 % par rapport à la dihydro-1,2 hydroxy-4 méthyl-5 oxo-2 pyridine), sur la cyclohexanone portant éventuellement le substituant R en position 2, on obtient l'hydrazone de formule (II) :

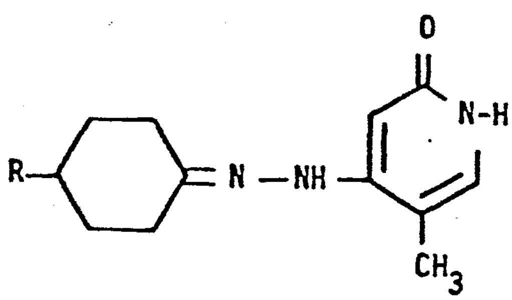

(II)

dans laquelle R a la même signification que dans la formule I,

2) on cyclise l'hydrazone de formule (II) selon la réaction de FISHER pour former l'hexahydro-1, 2, 6, 7, 8, 9 méthyl-4 oxo-1 5-H pyrido (4,3-b) indole de formule (III) :

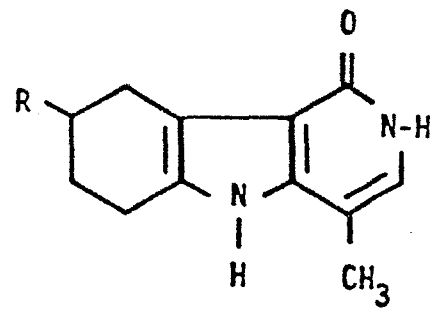

(III)

dans laquelle a la même signification que dans la formule I, et

3) on déshydrogène l'hexahydro pyrido indole de formule (III) par action du charbon palladié pour former le dihydro-1,2 méthyl-4 oxo-1 5-H pyrido (4,3-b) indole de formule (I).

La formation de l'hydrazone (II) s'effectue à la température de reflux d'un solvant polaire tel que l'éthanol.

La cyclisation de l'hydrazone (II) est obtenue par chauffage à des températures comprises entre 200°C et 280°C au reflux d'un solvant inerte, tel que le diphényléther, en l'absence de catalyseur.

L'aromatisation du composé de formule (III), qui conduit à l'oxo-1 pyrido indole de formule (I), s'effectue dans le même solvant que celui mis en oeuvre dans l'étape précédente, à haute température et en présence d'un catalyseur tel que le charbon palladié, de telle sorte que l'isolement intermédiaire du composé (III) n'est pas indispensable.

Certains composés de formule (I) peuvent aussi être préparés comme suit :

1°) Par réaction de l'hydrazino-4 méthyl-5 oxo-2 dihydro- 1,2 pyridine, préparée selon la méthode décrite par E. BISAGNI (demande de brevet Français N° 85.04.871 du 22 Mars 1985), sur la diméthyl-3,3 dioxa-1,5 spiro

/5,5/ undécanone, ou tout autre monoacétal de la cyclohexanedione-1,4 préparée par exemple selon la méthode de J. M. KAMENKA /Bull. Soc. Chim. France, 3,4 87-88, 1983/, on obtient l'hydrazone de formule :

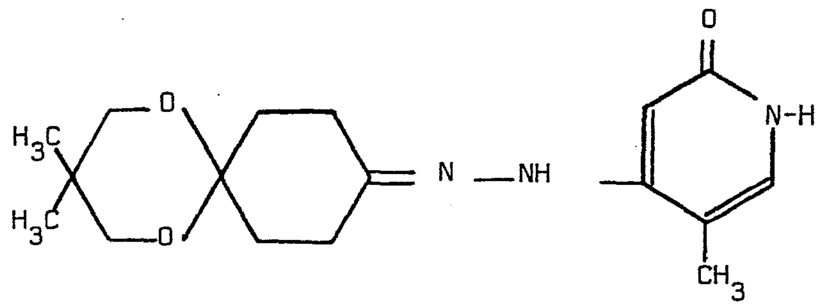

(IV)

2°) L'hydrazone de formule (IV) est cyclisée selon la réaction de FISHER, et le dérivé cyclique obtenu hydrolysé pour former la méthyl-4 tétrahydro-6,7,8,9 2-H 5-H pyrido /4,3-b/ indoledione-1,8 de formule :

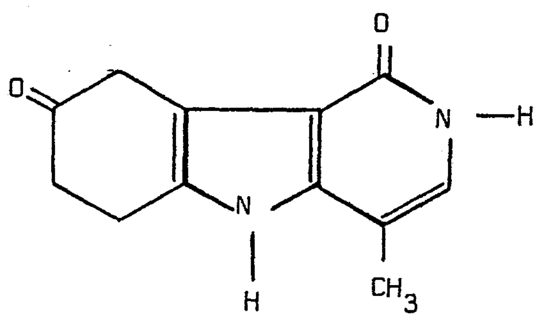

(V)

3°) La pyrido-indoledione de formule (V) est déshydrogénée par action du charbon palladié pour former l'hydroxy-8 méthyl-4 2-H pyrido /4,3-b/ indolone-1 de formule :

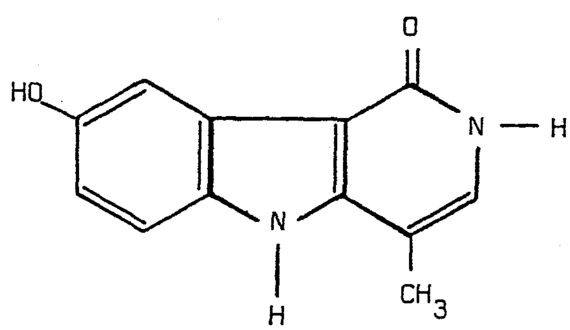

(VI)

4°) Le dérivé hydroxylé (VI) est estérifié par action d'un anhydride d'acide aromatique pour former une arylcarbonyloxy-8 méthyl-4 2-H 5-H pyrido /4,3-b/ indolone-1 de formule I dans laquelle $R_2$ représente le grou-

pe aryl($C_6$-$C_{10}$)carbonyloxy :

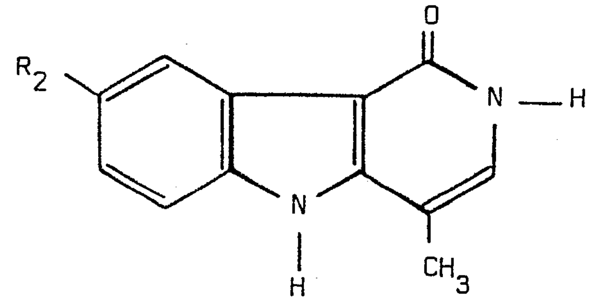

(I)

La formation de l'hydrazone de formule (IV) s'effectue à la température de reflux d'un solvant polaire tel que l'éthanol.

La cyclisation du composé de formule (IV) est obtenue par chauffage à des températures comprises entre 200°C et 280°C au reflux d'un solvant inerte tel que le diphényl éther.

Après hydrolyse par une solution aqueuse normale d'acide chlorhydrique portée au reflux, l'aromatisation du composé de formule (V), qui conduit au composé de formule (VI) s'effectue dans le même solvant que celui mis en oeuvre dans l'étape précédente, à haute température et en présence d'un catalyseur tel que le charbon palladié.

L'estérification du composé de formule (VI) est effectuée à la température de reflux d'un solvant basique aprotique tel que la pyridine.

Les exemples suivants sont donnés à titre d'illustration de la présente invention :

EXEMPLE 1

Préparation du dihydro-1,2 méthoxy-8 méthyl-4 oxo-1 5-H pyrido (4,3-b) indole (composé de formule (I) avec R = $OCH_3$).

A) méthoxy-4 [(dihydro-1,2 méthyl-5 oxo-2 pyridyl-4) hydrazono]-1 cyclohexanne (composé de formule (II) avec R = $-OCH_3$).

Dans 500 ml d'éthanol absolu on introduit 25 g (0,18 mole) de dihydro-1,2 hydrazino-4 méthyl-5 oxo-2 pyridine puis 25 g (0,195 mole) de méthoxy-4 cyclohexanone, qui a été préparée selon la méthode de J. W. COOK et al. (J. Chem. Soc., 322-324, 1944).

Le milieu est porté au reflux et après être passé par une phase homogène, il devient hétérogène car l'hydrazone (II) attendue précipite progressivement. Après 4 heures de reflux, on élimine par distillation à pression ordinaire, 250 ml d'éthanol. Puis le milieu est refroidi à 0°C. Le précipité formé est filtré, lavé à l'éther éthylique et séché.

On obtient 38,8 g d'hydrazone (II).

Rendement : 86 % ; F : 270°C

Calculé pour C13 H19 N3 O2 : C, 62,68 ; H, 7,69 ; N, 16,87 ;
trouvé : C, 62,79 ; H, 7,84 ; N, 16,84.

B) Hexahydro-1, 2, 6,7, 8, 9 méthoxy-8 méthyl-4 oxo-1 5-H pyrido (4,3-b) indole (composé de formule (III) avec R = -OCH3).

Sous agitation et sous atmosphère d'azote, on porte 450 ml de diphényléther à la température de 80°C. Puis on ajoute 20 g (0,08 mole) d'hydrazone précédente et le milieu est chauffé au reflux que l'on maintient pendant 30 minutes. Après refroidissement à 50°C, 400 ml de toluène sont introduits dans le milieu qui est ensuite refroidi à 15°C. Le précipité obtenu est filtré, lavé à l'acétone puis à l'éther éthylique. On isole ainsi 17,7 g d'hexahydropyrido-indole attendu.

Rendement : 95 % ; F > 270°C

Calculé pour C13 H16 N2 O2 : C, 67,22 ; H, 6,94 ; N, 12,06 ;
trouvé : C, 67,12 ; H, 6,95 ; N, 12,16.

RMN 1H (CD5N) ; $\delta$ : 1,85-2,07 (m, 2H, CH2-7) ; 2,21 (s, 3H, CH3-4) ; 2,66-2,94 (m, 2H, CH2-6) ; 3,35 (s, 3H, O-CH3) ; 3,57-3,93 (m, 3H, H-8 + CH2-9) ; 6,94 (s, 1H, H-3) ; 11,54 (s, 1H, NH-2); 11,94 (s, 1H, NH-5).

C) Dihydro-1,2 méthoxy-8 méthyl-4 oxo-1 5-H pyrido (4,3-b) indole. Dans 2 litres de diphényléther, on introduit 30 g de charbon palladié à 10 %. Puis, sous agitation et sous atmosphère d'azote, on porte le milieu à la température de reflux. On ajoute alors progressivement 134 g (0,58 mole) d'hexahydropyrido-indole obtenu selon l'étape B et l'on maintient au reflux le milieu pendant 1 heure environ après cette addition jusqu'à la fin du dégagement d'hydrogène. Après avoir refroidi à 50°C, on introduit 1 litre de toluène et l'on refroidit le milieu à 15°C. Le précipité formé est filtré puis recristallisé dans 1,5 litre d'acide acétique. Le produit pur ainsi obtenu est filtré, lavé à l'acétone puis à l'éther éthylique. On isole 97 g d'oxo-1 pyrido-indole attendu pur. La solution d'acide acétique est concentrée sous pression réduite. Le précipité formé est filtré, lavé à l'acétone puis à l'éther éthylique. On isole ainsi de nouveau 15,6 g d'oxo-1 pyrido-indole attendu pur.

Rendement : 86 % ; F $>$270°C

Calculé pour C13 H12 N2 O2 : C, 68,45 ; H, 5,30 ; N, 12,28 ;

trouvé : C, 68,26 ; H, 5,15 ; N, 12,27.

RMN 1H [ (CD3)2SO ] ; $\delta$ : 2,26 (s, 3H, CH3-4) ; 3,84 (s, 3H, OCH3) ; 6,94 (q, 1H, H-7, J 7-6 = 8,6 Hz, J 7-9 = 2,8 Hz) ; 7,07 (s, 1H, H-3) ; 7,43 (d, 1H, H-6) ; 7,65 (d, 1H, H-9) ; 10,82 (s, 1H, NH-5) ; 11,55 (s, 1H, NH-2).

EXEMPLE 2

Préparation du dihydro-1,2 méthoxy-8 méthyl-5 oxo-1 5-H pyrido (4,3-b) indole (composé de formule (I) avec R = -OCH3) sans isoler le composé de formule (III).

Dans 200 ml de diphényl éther, on introduit 8,1 g (0,058 mole) d'hydrazone préparée selon la méthode décrite dans l'étape A de l'exemple 1. Puis sous agitation et sous atmosphère d'azote on porte le milieu au reflux qui est maintenu pendant 30 minutes. Après refroidissement vers 100°C on ajoute ensuite, sous atmosphère d'azote, une suspension de 2 g de charbon palladié à 10 % dans 30 ml de diphényléther. Le milieu est alors chauffé au reflux pendant 30 minutes. Après refroidissement à 60°C, on introduit 400 ml d'éthanol et le milieu est chauffé à reflux.

Le charbon palladié est filtré à chaud puis lavé par 100 ml d'éthanol bouillant.

Ce filtrat éthanolique est ajouté au milieu diphényléther-éthanol qui est ensuite concentré sous pression réduite pour éliminer l'éthanol. A la phase diphényléther on ajoute 200 ml de toluène. Le précipité obtenu est filtré puis recristallisé dans l'éthanol. On obtient ainsi 5,9 g d'oxo-1 pyrido-indole attendu.

Rendement : 79,7 % ; F > 270°C

Le produit obtenu possède les mêmes caractéristiques (IR ; RMN) que l'échantillon préparé selon la méthode N°1 de l'exemple 1.

A titre illustratif, on décrit ci-dessous la préparation d'un composé de formule (A) à partir d'un composé de formule (I) :

Préparation de (diéthylamino-3 propylamino)-1 diméthyl-4,5 méthoxy-8 5-H pyrido (4,3-b) indole. (composé de formule (A) avec R1 = -CH3, R2 = -OCH3, R3 = R4 = -C2H5, R5 = H, et n = 3).

A) chloro-1 méthyl-4 méthoxy-8 5-H pyrido (4,3-b) indole

7 g du dihydro-1,2 méthoxy-8 méthyl-4 oxo-1 5-H pyrido (4,3-b) indole sont chauffés dans l'oxychlorure de phosphore (200 ml) à reflux pendant 3 jours et l'excès d'oxychlorure est évaporé sous pression réduite. Le résidu est repris dans 100 ml d'eau bouillante, le mélange est chauffé à l'ébullition pendant 3 minutes et filtré. Le filtrat est alcalinisé à froid par l'ammoniaque et le précipité formé est essoré, séché puis recristallisé dans l'acétonitrile pour donner 5,8 g de microcristaux jaunes.

Rendement : 76 % ; F : 243-245°C

Calculé pour C13 H11 Cl N2O : C, 63,29 ; H, 4,49 ; N, 11,35 ; Cl, 14,37 ;

trouvé : C, 63,02 ; H, 4,38 ; N, 11,28 ; Cl, 14,55

RMN H1 [(CD3) 2SO] ; $\delta$ : 2,52 (d, 3H, CH3-4, JCH3-H-3 = 1Hz) ; 3,91 (s, 3H, OCH3) ; 7,23 (q, 1H, H-7, J 7-6 = 9Hz, J 7-9 = 2,5 Hz) ; 7,60 (d, 1H, H-6) ; 7,87 (d, 1H, H-9) ; 8,04 (d, 1H, H-3).

B) chloro-1 diméthyl-4,5 méthoxy-8 5-H pyrido (4,3-b) indole

On dissout 20 g de chloro-1 méthoxy-8 méthyl-4 5-H pyrido (3,2-b) indole (0,081 mole) dans 300 ml de N,N diméthylformamide anhydre, puis on introduit 86 g de carbonate de potassium (0,6 mole) et 25 g d'iodure de méthyle (0,178 mole) à la température de 20°C sous agitation maintenue pendant 24 heures.

Le précipité est filtré. Puis la solution est concentrée sous pression réduite. Le second précipité est filtré. Les deux précipités sont alors dissous dans l'eau bouillante acidifiée par une solution aqueuse d'acide chlorhydrique N. La solution aqueuse refroidie est ensuite neutralisée par une solution d'ammoniaque diluée. Le précipité obtenu est filtré, lavé à l'eau, séché et recristallisé dans l'éthanol. On obtient 15 g du produit attendu.

Rendement : 71 % ; F : 180°C

Calculé pour C14 H13 Cl NO2 : C, 64,49 ; H, 5,03 ; N, 10,74 ; Cl, 13,60

trouvé : C, 64,73 ; H, 5,01 ; N, 10,52 ; Cl, 13,32

RMN H1 [(CD3)2SO] ; δ : 2,28 (d, 3H, CH3-4, J CH3-H-3 = 1Hz) ; 3,91 (s, 3H, OCH3) ; 4,14 (s, 3H, NCH3) ; 7,29 (q, 1H, H-7, J 7-6 = 8,9 Hz, J 7-9 = 2,5 Hz) ; 7,70 (d, 1-H, H-6) ; 7,91 (d, 1H, H-9) ; 8 (d, 1H, H3).

C) (Diéthylamino-3 propylamino)-1 diméthyl-4,5 méthoxy-8 5-H pyrido (4,3-b) indole

Ce composé est préparé par action du chloro-1 diméthyl-4,5 méthoxy-8 5-H pyrido (4,3-b) indole (1 g) sur la diéthylamino-3 propylamine (20 ml) portée au reflux pendant 44 heures. Puis l'excès d'amine est distillé sous pression réduite. Le résidu est repris dans 20 ml d'eau, alcalinisé par une solution aqueuse normale d'hydroxyde de sodium et enfin extrait par le chlorure de méthylène. La phase organique est séchée, puis concentrée sous pression réduite. Le produit brut ainsi obtenu est purifié par chromatographie sur colonne d'alumine en éluant tout d'abord par le chlorure de méthylène puis par le mélange chlorure de méthylèneméthanol (98/2).

Rendement : 85 % ; F : 107-109°C,
RMN H1 /($(CD_3)$ 2SO/ ; : 0,99 (t, 2 x 3H, CH3-CH2) ; 1,81 (m, 2H, CH2-B) ; 2,50-2,54 (m, 3 x 2H, (CH2-CH3) + CH2-γ); 2,62 (d, 3H, CH3-4, J CH3-H-3 = 0,7 Hz) ; 3,60 (m, 2H, CH2-α ) ; 3,90 (s, 3H, OCH3) ; 4,05 (s, 3H, NCH3) ; 6,35 (t, 1H, NH) ; 7,09 (q, 1H, H-7, J 7-6 = 9 Hz, J 7-9 = 2,3 Hz) ; 7,52 (d, 1H, H-6) ; 7,69 (d, 1H, H-3) ; 7,74 (d, 1H, H-9).

EXEMPLE 3

Préparation du benzoyloxy-8 méthyl-4 5-H pyrido [4,3-b] indole (composé de formule (I) dans lequel R = $C_6H_5$-CO-O).

A) Diméthyl-3,3 dioxa-1,5 [(méthyl-5 oxo-2 dihydro-1,2 1-H pyridyl-4) hydrazono]-9 spiro[5,5]undécane.

Le mélange formé par l'hydroxy-4 méthyl-5-1H pyridone-2 (16 g), l'éther monoéthylique de l'éthylène glycol (400 ml) et l'hydrate d'hydrazine (140 ml) est chauffé au reflux pendant 4 jours et évaporé à sec sous pression réduite. Le résidu solide est repris dans 500 ml d'éthanol absolu bouillant, filtré et le filtrat est concentré de moitié.

Après une nuit à la température ambiante, le solide cristallisé est essoré et séché. On obtient des cristaux incolores (13 g) correspondant à l'hydrate du composé recherché F = 135-155°.

Calc. pour $C_6H_9N_3O,H_2O$ = C, 45,85 ; H, 7,05 ; N, 26,74.
Trouvé : C, 45,29 ; H, 6,97 ; N, 26,03.

Les eaux mères concentrées à 100 ml et abandonnées une nuit à la température ambiante fournissent une nouvelle quantité du composé (2,4 g). Le rendement total s'élève donc à 15,4 g, soit 76 p. 100.

Le mélange de l'hydrate de l'hydrazino-4 méthyl-5 1-H pyridone-2 (3,56 g) et de la diméthyl-3,3 dioxa-1,5 spiro [5,5] undécanone-9 (7,92 g) dans l'éthanol absolu (200 ml) est chauffé à reflux pendant 2 h 15, puis refroidi à la température ambiante. Le solide filtré est lavé à l'éthanol pour donner 9 g de l'hydrazone pure. L'évaporation des eaux mères laisse un résidu solide qui

est repris dans 100 ml de dioxane bouillant, refroidi à l'ambiante, filtré puis recristallisé dans l'éthanol en donnant 1,2 g de produit attendu.

Rendement : 90,3 % ; F 260°C.

Calc. pour $C_{17}H_{25}N_3O_3$ : C, 63,92 ; H, 7,89, N, 13,16.

Trouvé : C, 63,64 ; H, 7,66, N, 12,84.

B) Tétrahydro-6,7,8,9 méthyl-4 2-H 5-H pyrido /4,3-b/ indoledione-1,8.

Le mélange formé par l'hydrazone précédente (2,3g) dans le diphényléther (45ml) est chauffé à reflux sous atmosphère d'argon pendant 40 minutes et laissé refroidir à la température ambiante.

Après addition de 150 ml de xylène, le précipité formé est essoré et lavé au xylène pour donner 2g de l'intermédiaire brut, puis hydrolysé par chauffage à l'ébullition pendant 25 minutes dans 100 ml d'acide chlorhydrique N, refroidi et alcalinisé par addition de carbonate de potassium. Le solide obtenu est filtré, séché et recristallisé dans le minimum d'éthanol pour donner 1,1g de microcristaux incolores correspondant à l'hydrate de l'indoledione attendue.

Rendement : 70,6 % ; F >260°C.

Calc. pour $C_{12}H_{12}N_2O_2$; $H_2O$ : C, 61,53 ; H, 5,98 ; N, 11,96.

Trouvé : C, 61,15 ; H, 5,87 ; N, 11,73.

RMN $H_1$ /$(CD_3)_2SO$/ ; $\delta$ : 2.15 (s,3H,$CH_3$-4), 2.68 (t,2H,$CH_2$-7), 3.06 (t, 2H,$CH_2$-6), 3.61 (s,2H,$CH_2$-9), 6.71 (s large, 1H,H-3), 10.4 (s large, 1H,NH -5), 11.23 (s large, 1H,NH-2).

C) Hydroxy-8 méthyl-4 2-H 5-H pyrido /4,3-b/ indolone-1.

Le mélange de la cétone précédemment obtenue (1,37 g) et de charbon palladié à 10% (1 g) dans le diphényléther (100 ml) est chauffé à reflux, sous agitation, pendant 30 minutes et refroidi à la température ambiante. On ajoute 400 ml d'éthanol, on filtre, on lave le solide avec 200 ml d'éthanol bouillant et évapore l'éthanol sous pression réduite. Après addition de 150 ml de toluène au diphényléther résiduel, le précipité obtenu est filtré, séché et repris dans le dioxane bouillant contenant la quantité

suffisante d'éthanol pour le dissoudre.

La solution filtrée est concentrée pour éliminer l'éthanol et refroidie pour fournir des microcristaux beige-clair (1,1 g).

Rendement : 81 % ; F > 260°C.

Calc. pour $C_{12}H_{10}N_2O_2$ : C, 67,28 ; H, 4,71 ; N, 13,08.
Trouvé : C, 66,89 ; H, 4,74 ; N, 12,84.

RMN $H_1$ [$(CD_3)_2SO$] ; δ : 2.25 (s,3H,$CH_3$-4), 6.8 (q,1H,H-7, J 7-6 = 9 Hz, J 7-9 = 2,3 Hz), 7.07 (m,1H,OH-8), 7.32 (d,1H,H-6), 7.56 (d,1H,H-9), 8.92 (s,1H,H-3), 10.09 (s large, 1H,NH-5), 11.4 (s large, 1H,NH-2).

D) Benzoyloxy-8 méthyl-4 2-H 5-H pyrido [4,3-b] indolone-1.

La pyrido indolone précédente (4 g) est traitée avec de l'anhydride benzoïque en excès (12,6 g) dans la pyridine (100 ml) à reflux pendant 2 h 15 et la pyridine est évaporée. Le résidu est repris dans une solution de carbonate acide de sodium en excès et la phase aqueuse est décantée après 1 heure sous agitation. A la fraction insoluble, on ajoute 50 ml d'éthanol, et le précipité obtenu est essoré, lavé à l'éthanol froid et repris dans l'éthanol bouillant (100 ml) pendant 5 minutes.

Le mélange refroidi fournit 4,4 g de microcristaux beige-clair.

Rendement : 74 % ; F > 260°C.

Calc. pour $C_{19}H_{14}N_2O_3$ : C, 71,69 ; H, 4,43 ; N, 8,80.
Trouvé : C, 71,32 ; H, 4,39 ; N, 8,81.

RMN $H_1$ [$(CD_3)_2SO$] ; δ : 2.31 (s,3H,$CH_3$-4), 7.16 (s,1H,H-3), 7.24 (q,1H,H-7, J 7-6 = 8,5 Hz J 7-9 = 1,8 Hz), 7.6 (d,1H,H-6), 7.73 (m,3H de $C_6H_5$), 7.96 (d,1H,H-9), 8.23 (m,2H de $C_6$-$H_5$), 10.96 (s large, 1H,NH-2), 11.92 (s large, 1H,NH-5).

## REVENDICATIONS

1. Composés de formule (I)

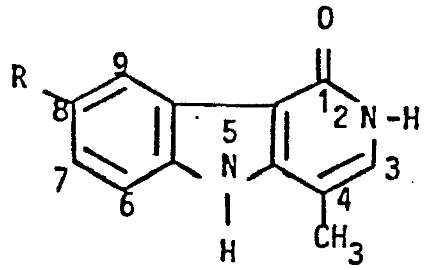

(I)

dans laquelle R représente l'hydrogène ou un groupe alkoxy en $C_1$-$C_4$ ou aryl ($C_6$-$C_{10}$)carbonyloxy

2. Procédé de préparation des composés (I) selon la revendication 1, caractérisé en ce que :

a) on fait réagir la dihydro-1,2 hydrazino-4 méthyl-5 oxo-2 pyridine sur la cyclohexanone portant éventuellement le substituant en position 4 pour obtenir l'hydrazone de formule (II)

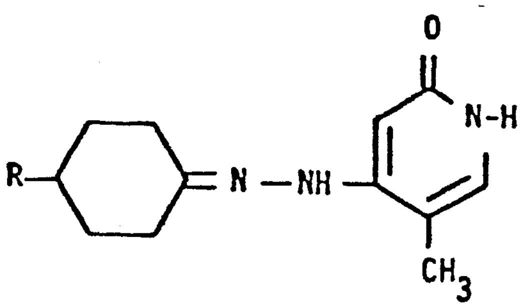

(II)

dans laquelle R a la même signification que dans la formule I,

b) on cyclise l'hydrazone de formule (II) pour obtenir une hexahydro -1, 2, 6, 7, 8, 9 méthyl-4 oxo-1 5-H pyrido (4,3-b) indole de formule (III)

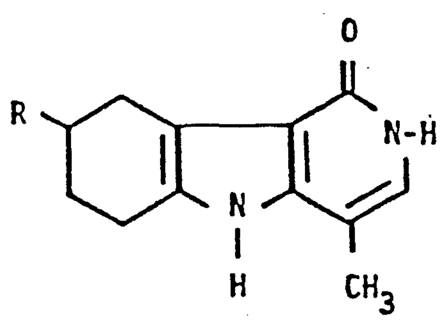

(III)

dans laquelle R a la même signification que dans la formule I,

c) on déshydrogène l'hexahydro pyrido-indole de formule (III) pour obtenir le dihydro-1,2 méthyl-4 oxo-1 5-H pyrido (4,3-b) indole de formule (I).

3. Procédé selon la revendication 2, caractérisé en ce que selon une variante, la déshydrogénation du composé de formule (III) est effectuée sans isolement préalable.

4. Procécé selon la revendication 2, caractérisé en ce que la cyclisation de l'hydrazone de formule (II) en hexahydro pyrido-indole de formule (III) s'effectue en l'absence de catalyseur, à une température comprise entre 200° et 280°C.

5. Procédé selon les revendications 2 ou 3, caractérisé en ce que la déshydrogénation du composé de formule (III) s'effectue en présence de catalyseur tel que le charbon palladié, à une température comprise entre 200° et 280°C.

REVENDICATIONS

pour les Etats contractants AT, ES, GR.

1. Procédé de préparation de composés de formule (I)

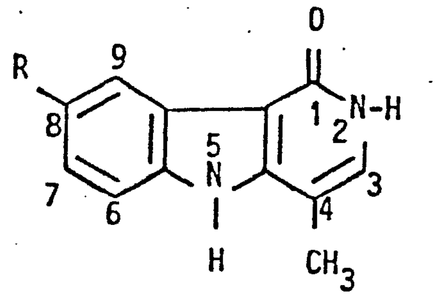

(I)

dans laquelle R représente l'hydrogène ou un groupe alkoxy en $C_1$-$C_4$ ou aryl ($C_6$-$C_{10}$)carbonyloxy, procédé caractérisé en ce que :

a) on fait réagir la dihydro-1,2 hydrazino-4 méthyl-5 oxo-2 pyridine sur la cyclohexanone portant éventuellement le substituant en position 4 pour obtenir l'hydrazone de formule (II)

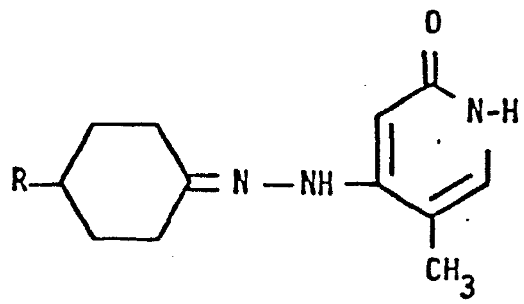

(II)

dans laquelle R a la même signification que dans la formule I,

b) on cyclise l'hydrazone de formule (II) pour obtenir une hexahydro-1, 2, 6, 7, 8, 9 méthyl-4 oxo-1 5-H pyrido (4,3-b) indole de formule (III)

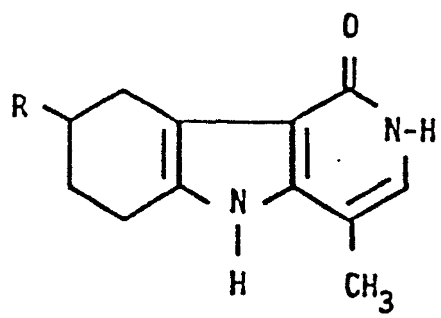

(III)

dans laquelle R a la même signification que dans la formule I,

c) on déshydrogène l'hexahydro pyrido-indole de formule (III) pour obtenir le dihydro-1,2 méthyl-4 oxo-1 5-H pyrido (4,3-b) indole de formule (I).

2. Procédé selon la revendication 1, caractérisé en ce que selon une variante, la déshydrogénation du composé de formule (III) est effectuée sans isolement préalable.

3. Procédé selon la revendication 1, caractérisé en ce que la cyclisation de l'hydrazone de formule (II) en hexahydro pyrido-indole de formule (III) s'effectue en l'absence de catalyseur, à une température comprise entre 200° et 280°C.

4. Procédé selon les revendications 1 ou 3, caractérisé en ce que la déshydrogénation du composé de formule (III) s'effectue en présence de catalyseur tel que le charbon palladié, à une température comprise entre 200° et 280°C.

Office européen des brevets

# RAPPORT DE RECHERCHE EUROPEENNE

Numéro de la demande 0238411

EP 87 40 0594

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | JOURNAL OF HETEROCYCLIC CHEMISTRY, vol. 12, no. 5, octobre 1975, pages 963-967, Prouv, Utah, US; C. DUCROCQ et al.: "Azaindoles. V. Pyrido[4,3-b]indoles (gamma-carbolines) fonctionnalisés sur leur sommet 4: synthèse en une seule étape" * En entier * | 1,2 | C 07 D 471/04 // (C 07 D 471/04 C 07 D 221:00 C 07 D 209:00 ) |
| | ----- | | |
| | | | **DOMAINES TECHNIQUES RECHERCHES (Int. Cl.4)** |
| | | | C 07 D 471/00 |

Le présent rapport de recherche a été établi pour toutes les revendications

| Lieu de la recherche | Date d'achèvement de la recherche | Examinateur |
|---|---|---|
| LA HAYE | 18-06-1987 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82